# EUROPEAN PATENT APPLICATION

(11) **EP 4 806 794 A1**
(43) Date of publication of application: **16.09.2026**
(21) Application number: 26159018.6
(22) Date of filing: 17.02.2026
(51) Int. Cl.: B65B 7/28, B65B 55/02, B65B 55/10, A61L 2/10, B67C 3/22, B67C 7/00

(54) **AN ASEPTIC APPARATUS AND PROCESS FOR FILLING AND CLOSING CANS**

(30) Priority: 11.03.2025 IT 202500005004
(71) Applicant: Gea Procomac S.p.A., 43038 Sala Baganza (PR) (IT)
(72) Inventor: COMANI, Andrea, 43038 SALA BAGANZA (PARMA) (IT); CALLEGARI, Fabio, 43038 SALA BAGANZA (PARMA) (IT); BELLINI, Vittorio, 43038 SALA BAGANZA (PARMA) (IT); CASAPPA, Luigi, 43038 SALA BAGANZA (PARMA) (IT)
(74) Representative: Dondi, Silvia

(57) **Abstract**

An aseptic apparatus (1) for filling and closing cans (100), the aseptic apparatus (1) comprising:
- a filling unit (2);
- a lid sterilizing unit (3) configured to sterilize lids (200) and comprising a rotating star-wheel, the lid sterilizing unit (3) being configured to lower each of the sterilized lids (200) on a corresponding can (100) laying on a conveyor (8) in a closing station (9) and to place and press the sterilized lid (200) on the corresponding can (100);
- isolating means (50, 60) within which the filling unit (2) and the sterilizing unit (3) are arranged;
- a seaming unit (10) arranged downstream the closing station (9) so as to receive the cans (100) with lids (200) applied thereon, the seaming unit (10) being located outside the isolating means (12).

## Description

The present invention relates to an aseptic apparatus and process for filling and closing cans.

Aseptic conditions are mandatory for "sensitive" food products, i.e., products which are particularly sensitive to bacteriological contamination and oxidation, such as isotonic beverages, juices, nectars, soft drinks, tea, milk-based beverages, coffee-based beverages, etc., for which avoiding possible microbiological contamination throughout all the packaging steps is of fundamental importance.

Document JP 3556063 B2 discloses an aseptic beverage can production facility where both the filling device and the seaming device are arranged in a clean chamber which maintains a level of air cleanliness in Class 10 to 100.

Nevertheless, it must be noticed that seaming devices are complex machines with a high number of moving parts, thus arranging them in the same sterile environment of the filler poses issues, especially linked to maintenance and format changes operations.

Document EP 0750562 B1 relates to an apparatus for aseptically filling and sealing cans. The installation comprises an empty can feed device, a can-filler device, a lid-dispenser device, and a crimping device for crimping on the lids. The filler device and the lid-dispenser device are arranged in an enclosure under a sterile atmosphere where sterile laminar flow is established. The crimping device is arranged in another enclosure where a turbulent sterile air flow is established. This specific arrangement of the crimping device allows to intervene on it, in particular for greasing its components without destroying the sterility conditions to which the filling device and the lid dispensing device must imperatively be subjected.

Nevertheless, the solution proposed in document EP 0750562 B1 does not address the issue of sterilizing lids and assuring they do not re-contaminate along the path to the crimping device.

Document WO 2018/099613 A1 discloses a lid separator for separating and treating lids, in particular for cleaning or sterilizing metallic lids used for closing containers like food and beverage cans.

A screw shaft rotating about an axis separates individual lids from a lid stream. Lids are arranged on the screw shaft at predetermined distances with gaps between them. A treatment device applies a treatment medium to the lids in a designated treatment area of the screw shaft.

This solution is structurally complex and time consuming.

In fact, the lids need to be separated for being sterilized. Then, the sterilized lids need to be brought closer again and packed to be fed in line to a dispensing device which is able to dispense them to the cans. All these operations are also affected by an elevated risk of recontamination of the lids before they actually arrived on the cans.

In this context, the technical task at the basis of the present invention is to propose an aseptic apparatus and process for filling and closing cans, which overcome the problems of the prior art cited above.

In particular, the object of the present invention is to provide an aseptic apparatus and process for filling and closing cans, which is able to guarantee a reliable sterilization of the lids to be applied to the cans, reducing the risks of recontamination of the prior art solutions.

Another object of the present invention is to propose an aseptic apparatus for filling and closing cans, which is more compact and structurally simpler than the prior art solutions.

The stated technical task and specified aims are substantially achieved by an aseptic apparatus for filling and closing cans, the aseptic apparatus comprising:
- a filling unit configured to receive the cans and to inject a product within the cans;
- a lid sterilizing unit configured to sterilize lids and comprising a rotating star-wheel having a plurality of pockets, each pocket of said plurality of pockets being adapted to receive one of the lids;
- a conveyor configured to advance the cans filled with product from the filling unit towards the lid sterilizing unit, said lid sterilizing unit being configured to lower each of the sterilized lids held in one of the pockets on a corresponding can laying on said conveyor in a closing station and to place and press said sterilized lid on the corresponding can;
- isolating means within which the filling unit and the sterilizing unit are arranged;
- a seaming unit arranged downstream the closing station so as to receive the cans with lids applied thereon.

According to one aspect of the invention, the seaming unit is located outside the isolating means.

According to one aspect of the invention, the conveyor extends towards the seaming unit so that a first tract of the conveyor is within the isolating means and a second tract of the conveyor is outside the isolating means. In particular, the rotating star-wheel of the lid sterilizing unit is arranged in a protected environment which is saturated with VHP.

According to one embodiment of the invention, the lid sterilizing unit comprises supporting means for supporting each lid in a loose condition within a corresponding pocket of the rotating star-wheel in such a way that a circular trajectory of the lid held in said loose condition in the pocket is tangent in the closing station to a line that is parallel to a linear advancing direction of the cans laying on the conveyor.

In other words, the lid held in loose condition in the pocket describes a circular trajectory that is tangent to a line parallel to the linear advancing direction of the cans.

According to another embodiment of the invention, the lid sterilizing unit comprises supporting means for supporting each lid in a loose condition within a corresponding pocket of the rotating star-wheel in such a way that the lid held in said loose condition the pocket describes a linear trajectory in approaching the closing station. In particular, the linear trajectory is parallel or tangent to a linear advancing direction of the cans laying on the conveyor.

Preferably, the supporting means for supporting each lid in a loose condition within a corresponding pocket comprise at least:
- an upper barrier configured to limit upper movements of each lid held in the corresponding pocket;
- a lower barrier configured to limit lower movements of each lid held in the corresponding pocket;
- an external barrier configured to limit lateral movements of each lid held in the corresponding pocket.

In particular, both the upper barrier and the lower barrier have a partially circular development which follows the circumferential development of the rotating star-wheel.

In one example, the upper barrier comprises two upper circular rods which are concentrically arranged with respect to a center of the rotating star-wheel, and the lower barrier comprises two lower circular rods which are concentrically arranged with respect to the center of the rotating star-wheel.

Preferably, the supporting means further comprise an internal barrier configured to abut each lid held in the corresponding pocket so as to force the lid to change its position.

According to one embodiment of the invention, the lid sterilizing unit comprises a pushing organ configured to lower each lid towards a corresponding can laying on the conveyor in the closing station.

According to another embodiment of the invention, the lid sterilizing unit comprises a pushing organ integrally fixed to the rotating star-wheel in correspondence of each pocket of the plurality of pockets. Each pushing organ is configured to lower a corresponding lid towards a can laying on the conveyor in the closing station.

Preferably, the lid sterilizing unit comprises dispensing means for dispensing a sterilizing substance. The dispensing means comprise first nozzles located at a higher level with respect to the pockets and second nozzles located at a lower level with respect to the pockets.

In particular, the first nozzles and the second nozzles are configured to dispense the sterilizing substance respectively towards top of the lids and bottom of the lids.

Alternatively, or in addition to chemical sterilization, the lid sterilizing unit is configured to sterilize the lids (200) by radiation.

According to one aspect of the invention, the aseptic apparatus further comprises actuating means configured to adjust a height of the rotating star-wheel with respect to a floor or ground.

According to one aspect of the invention, the aseptic apparatus further comprises an upper guide extending from the closing station to the seaming unit and configured to maintain the lids placed and pressed on the corresponding cans upon leaving the closing station.

Preferably, a sterile air flow is established over the upper guide in order to flow on the lids applied on the advancing cans.

Preferably, the aseptic apparatus further comprises a lid infeed device configured to supply the lids to the pockets of the rotating star-wheel.

In particular, the lid infeed device comprises a longitudinal guide for housing the lids in a stacked arrangement.

The stated technical task and specified aims are substantially achieved by a process for aseptically filling and closing cans using the aseptic apparatus according to the invention.

The process comprises at least the following steps:
- filling the cans with a product, the filling occurring in the filling unit;
- delivering lids to the pockets of the rotating star-wheel;
- sterilizing the lids in the lid sterilizing unit;
- making the cans to advance from the filling unit towards the lid sterilizing unit along a linear advancing direction on the conveyor;
- for each can arriving in the closing station, lowering one of the sterilized lids onto the can, then placing and pressing the sterilized lid on the can so as to close it;
- conveying the cans with the lids applied thereon towards the seaming unit;
- crimping the lids on the cans.

According to one embodiment of the invention, the sterilization of each lid in the sterilizing unit is at least partially overlapped to the step of placing lid onto the corresponding can in the closing station.

Preferably, lowering each sterilized lid comprises pushing the sterilized lid out of the corresponding pocket.

According to one embodiment of the invention, each lid on the rotating star-wheel follows a circular trajectory that is tangent in the closing station to a line that is parallel to the linear advancing direction of the cans.

According to another embodiment of the invention, each lid on the rotating star-wheel follows a linear trajectory in approaching the closing station. The linear trajectory is parallel or tangent to the linear advancing direction of the cans.

According to one aspect of the invention, the process further comprises sterilizing or washing the conveyor.

According to one aspect of the invention, the process further comprises adjusting a height of the rotating star-wheel with respect to a floor or ground.

Further characteristics and advantages of the present invention will more fully emerge from the non-limiting description of a preferred but not exclusive embodiment of an aseptic apparatus and process for filling and closing cans, as illustrated in the accompanying drawings, in which:

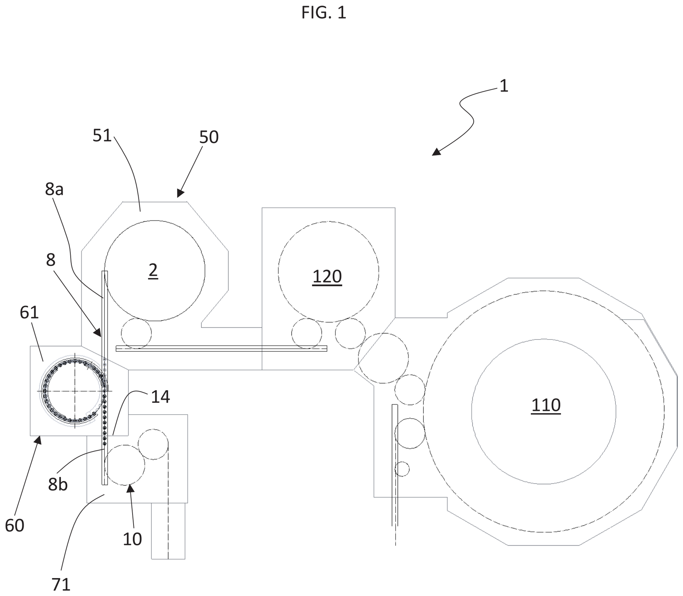

- figure 1 illustrates an aseptic apparatus for filling and closing cans, according to the present invention, in a plan view;
- figure 2 illustrates a part (lid sterilizing unit) of the aseptic apparatus of figure 1, in a plan view;

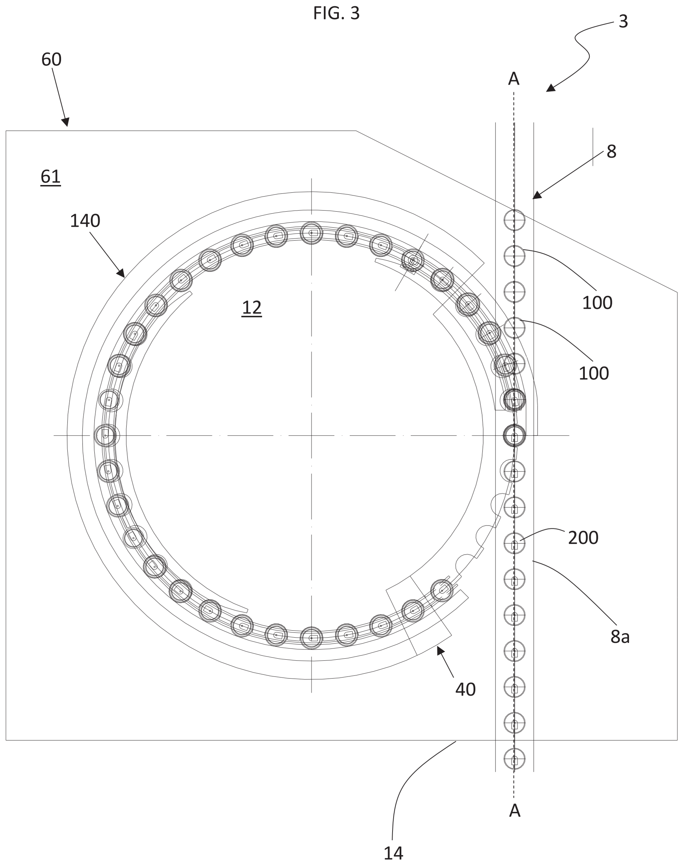

- figure 3 illustrates a part (lid sterilizing unit and part of the conveyor) of the aseptic apparatus of figure 1, in a plan view and in use (with cans and lids);
- figure 4 illustrates the lid sterilizing unit and conveyor of figure 4, in a lateral cross-sectional view;
- figure 5 illustrates a lid with supporting means and dispensing means, in a cross-sectional view;
- figure 6 illustrates a different embodiment of the lid sterilizing unit and conveyor, in a lateral view;
- figure 7 is an enlarged view of the lid sterilizing unit, with focus on a pocket with pushing organ, in a plan view;
- figure 8 illustrates a filled can with the lid maintained placed and press in the advancement towards the seaming unit.

With reference to the figures, number 1 denotes an aseptic apparatus for filling and closing cans 100.

In the following description, the aseptic apparatus will be shortly referred to as "apparatus".

According to an aspect of the invention, the apparatus 1 comprises a can sterilizing unit 110 configured to sterilize the cans 100. In particular, the cans 100 are chemically sterilized using a sterilizing agent.

The apparatus 1 further comprises a rising unit 120 arranged downstream the can sterilizing unit 110 with respect to an advancing path of the cans 100.

The apparatus 1 comprises a filling unit 2 configured to receive the cans 100 and to inject a product within the cans 100.

The filling unit 2 is arranged downstream the rinsing unit 120.The apparatus 1 further comprises a lid sterilizing unit 3 configured to sterilize lids 200 to be applied onto an open top 100a of each can 100.

The lid sterilizing unit 3 comprises a rotating star-wheel 4 having a plurality of pockets 5 which are arranged along a circumferential edge of the rotating star-wheel 4. Each pocket 5 is adapted to receive one of the lids 200.

In this context, a can 100 is a container made of thin metal, such as Aluminium or steel. In particular, a can 100 has a substantially cylindrical shape, with a circular open top 100a and a disk-shaped bottom 100b.

A lid 200 is made by metal and is shaped as a disk with a circular protuberance 200a to be crimped to the can 100.

In one embodiment of the invention, the lid sterilizing unit 3 is configured to chemically sterilize the lids 200. In particular, the lid sterilizing unit 3 comprises dispensing means 31, 32, 33, 34 for dispensing a sterilizing substance.

For example, the sterilizing substance is vaporized hydrogen peroxide. Alternatively, the sterilizing substance is peracetic acid.

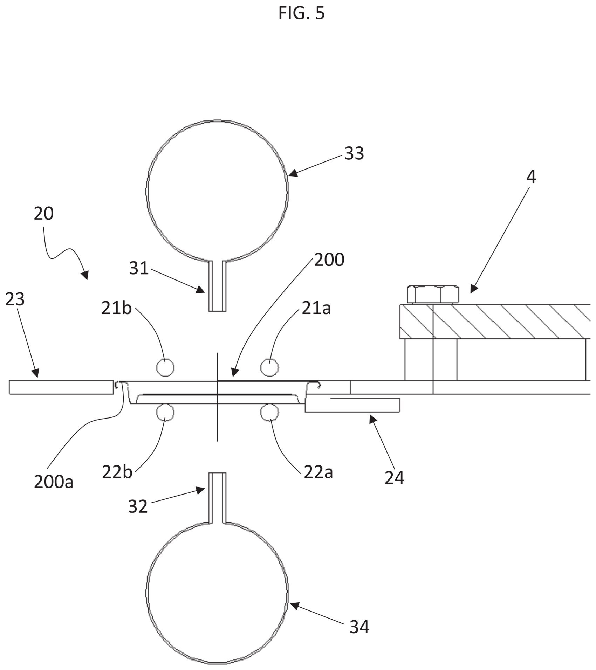

In one embodiment of the invention, shown in figure 5, the dispensing means comprises first nozzles 31 located at a higher level with respect to the pockets 5 (and thus to the lids 200) and second nozzles 32 located at a lower level with respect to the pockets 5 (and thus to the lids 200).

The first nozzles 31 and the second nozzles 32 are configured to dispense the sterilizing substance respectively towards the top of the lids 200 and the bottom of the lids 200.

In the illustrated embodiment, the first nozzles 31 and the second nozzles 32 receive the sterilizing agent from two different manifolds 33, 34. These manifolds 33, 34, which are also part of the dispensing means, are preferably in fluid communication.

According to another embodiment of the invention, the lid sterilizing unit 3 is configured to sterilize the lids 200 by radiation. For example, ionizing radiations are employed.

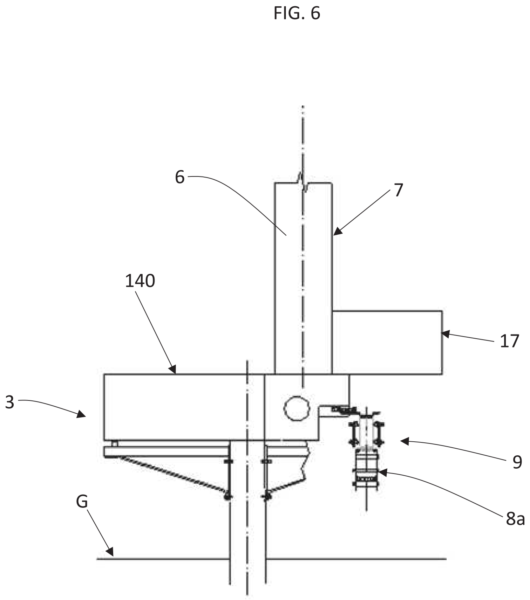

The lids 200 are supplied to the lid sterilizing unit 3 by means of a lid infeed device 6, shown in figure 6.

According to one example, the lid infeed device 6 comprises a longitudinal guide 7 for housing the lids 200 in a column configuration, which means that the lids 200 are stacked one on the other.

The lid infeed device 6 is located close to the lid sterilizing unit 3 so as to deliver the lids 200 to the pockets 5 of the rotating star-wheel 4.

In particular, the lid infeed device 6 further comprises an extracting device 17 for extracting a lid 200 from the bottom of the stack and for delivering the lid 200 to one of the pockets 5.

According to an alternative example, not shown, the lid sterilizing unit 3 comprises a device which is configured to pick a lid 200 and place it on a corresponding can 100. This pick and place device is similar to devices operating on capsules for closing bottles.

The apparatus 1 further comprises a conveyor 8 configured to advance the cans 100 filled with the product from the filling unit 2 towards the lid sterilizing unit 3.

In particular, the cans 100 are arranged to form a line on the conveyor 8 and to move along a linear advancing direction A-A. The linear advancing direction A-A preferably coincides with a longitudinal symmetry line of the conveyor 8.

The lid sterilizing unit 3 is arranged downstream the filling unit 2 with respect to the movement of the cans 100.

The lid sterilizing unit 3 is configured to apply a sterilized lid 200 held in one of the pockets 5 to a corresponding can 100 laying on the conveyor 8 in a closing station 9.

The lid sterilizing unit 3 is configured to lower a sterilized lid 200 on the corresponding can 100 in the closing station 9 and to place and press the sterilized lid 200 on the corresponding can 100, in particular for closing the open top 100a of the can 100.

In fact, the cans 100 advance along the linear advancing direction A-A at a level which is lower than the level of the rotating star-wheel 4 holding the lids 200.

In this context, with the term "level" is meant a height with respect to a floor or ground G on which the apparatus 1 is installed.

In the claimed invention, the sterilized lids 200 are lowered on the corresponding cans 100, which means that the lids 200 are moved along a substantially vertical direction from an initial position to a final lower position.

Lowering a sterilized lid 200 on the corresponding can 100 may be done according to different embodiments.

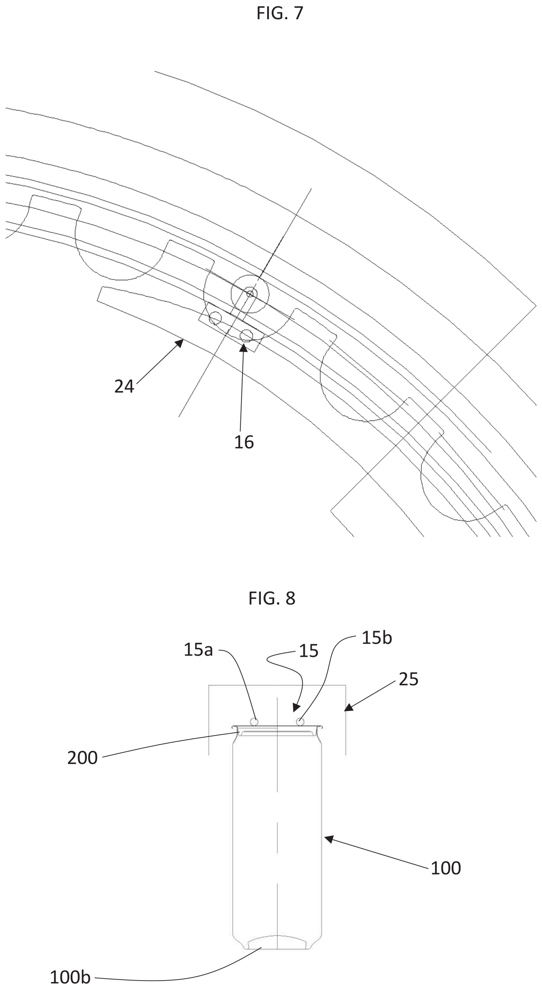

According to a first embodiment, shown in figure 7, the lid sterilizing unit 3 comprises a pushing organ 16 configured to lower each lid 200 towards the corresponding can 100 laying on the conveyor 8 in the closing station 9.

In particular, in this first embodiment there is only one pushing organ 16 which is in a stationary position with respect to the rotating star-wheel 4. This pushing organ 16 is configured to push a lid 200 out of the pocket 5 and to move the lid 200 towards the level of the open top 100a of the can 100 present in the closing station 9.

According to a second embodiment, the lid sterilizing unit 3 comprises a plurality of pushing organs (not shown) which are integrally fixed to the rotating star-wheel 4. Each pushing organ is arranged at one of the pockets 5 and is configured to lower the corresponding lid 200 towards the can 100 laying on the conveyor 8 in the closing station 9.

According to one aspect of the invention, the height of the rotating star-wheel 4 is adjustable. In particular, the apparatus 1 comprises actuating means (not shown) configured to adjust the height of the rotating star-wheel 4. This is advantageous in the event of can format changes.

Thus, the first or the second embodiments may be combined with this option so as to adjust the height of the rotating star-wheel 4 in case of a can format change.

The apparatus 1 further comprises a seaming unit 10 arranged downstream the closing station 9 so as to receive the cans 100 with lids 200 applied thereon.

The rotating star-wheel 4 of the lid sterilizing unit 3 is arranged in a protected environment 12, which is protected (i.e., shielded) by a box 140. Preferably, the protected environment 12 is a saturated VHP volume.

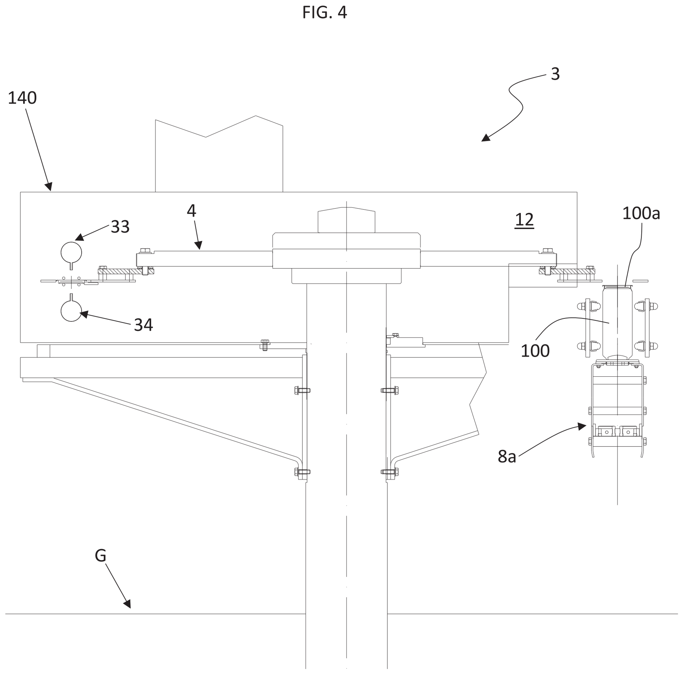

The dispensing means 31, 32, 33, 34 are also arranged within the protected environment 12, as shown in figure 4.

The lid infeed device 6 is located outside the protected environment 12. Preferably, an airlock system 40 is provided at the entrance and at the exit of the lid sterilizing unit 3.

As it is known, an airlock system is obtained by creating a differential pressure between two adjacent zones.

The filling unit 2 is arranged inside a first isolator 50 which delimits a first environment 51. Thus, the filling unit 2 is arranged within the first environment 51.

The box 140 is arranged inside a second isolator 60 which delimits a second environment 61. Thus, the box 140 is arranged within the second environment 61.

The first environment 51 and the second environment 61 are in fluid communication.

Preferably, the first environment 51 has a pressure level which is higher than the pressure level in the second environment 61. Alternatively, the first environment 51 and the second environment 61 are at the same pressure level.

The seaming unit 10 is located outside the protected environment 12. In particular, the seaming unit 10 is arranged within a third environment 71. The third environment 71 is an ultra-clean volume.

The pressure level within the third environment 71 is lower than the pressure level within the second environment 61.

Having a higher pressure level in the first environment 51, then an intermediate pressure level in the second environment 61 and finally a lower pressure level in the third environment 71 results in an air flow always directed from the filling unit 2 toward the seaming unit 10.

The same occurs also in the alternative solution in which the pressure level is the same in the first environment 51 and in the second environment 61, but in any case, higher than in the third environment 71.

In one example, the third environment 71 has a lower level of sterility with respect to the first and second environments 51, 61. The conveyor 8 extends to arrive to the seaming unit 10, thus it has a first tract 8a which is arranged within the first and the second environments 51, 61 and a second tract 8b which is arranged in the third environment 71.

In particular, the first tract 8a of the conveyor 8 extends from the filling unit 2 to an interface 14 between the second environment 61 and the third environment 71.

The second tract 8b of the conveyor 8 extends from said interface 14 to the seaming unit 10.

The conveyor 8 may be sterilized, for example using peracetic acid. Alternatively, the conveyor 8 may be washed or decontaminated according to methods known in the art.

Preferably, the apparatus 1 comprises an upper guide 15 extending from the closing station 9 to the seaming unit 10.

The upper guide 15 is configured to maintain the lids 200 placed and pressed on the corresponding cans 100 upon leaving the closing station 9, while advancing from the closing station 9 to the seaming unit 10.

In one example, shown in figure 8, the upper guide 15 comprises two rods 15a and 15b. In an alternative example (not shown), the upper guide 15 comprises a plate. Preferably, the upper guide 15 can be made in steel or Teflon.

Preferably, a sterile air flow is established between a cover 25 and the upper guide 15. The sterile air flow is configured to flow on the lids 200 placed of the advancing cans 100.

The lid sterilizing unit 3 comprises supporting means 20 for supporting each lid 200 in a loose condition within a corresponding pocket 5 of the rotating star-wheel 4.

In this context, the expression "loose condition" means that the lid 200 is arranged in the corresponding pocket 5 with freedom to move within a pre-established volume, and interdiction to move outside the pre-established volume. In other words, the lid 200 is not in a fixed position within the pocket 5, but it can change its position within the pre-established volume.

In one embodiment of the invention, each lid 200 is maintained within the corresponding pocket 5 by the supporting means 20 in such a way that a circular trajectory of the lid 200 held in said pocket 5 is tangent in the closing station 9 to a line that is parallel to the linear advancing direction A-A of the can 100.

In other words, a projection of the circular trajectory of the lid 200 on a plane that contains the conveyor 8 is tangent to the linear advancing direction A-A.

In another embodiment, each lid 200 is maintained within the corresponding pocket 5 by the supporting means 20 in such a way that the lid 200 held in said pocket 5 describes a linear trajectory in approaching the closing station 9. The linear trajectory is parallel or tangent to the linear advancing direction A-A.

According to a preferred example, shown in figure 5, the supporting means 20 comprise at least:
- an upper barrier 21a, 21b configured to limit upper movements of each lid 200 held in the corresponding pocket 5;
- a lower barrier 22a, 22b configured to limit lower movements of each lid 200 held in the corresponding pocket 5;
- an external barrier 23 configured to limit lateral movements of each lid 200 held in the corresponding pocket 5.

In particular, the upper barrier 21a, 21b is arranged at a higher level than the plane of the pockets 5 so as to limit upper movements of each lid 200 within the pocket 5.

In particular, the lower barrier 22a, 22b is arranged at a lower level than the plane of the pockets 5 for supporting each lid 200 and thus limiting lower movements of each lid 200 within the pocket 5.

In particular, the external barrier 23 is arranged around and out of the rotating star-wheel 4 so as to prevent the lids 200 from laterally exiting out of the pockets 5.

In particular, the upper barrier 21a, 21b has a partially circular development which follows the circumferential development of the rotating star-wheel 4.

Analogously, the lower barrier 22a, 22b has a partially circular development which follows the circumferential development of the rotating star-wheel 4.

The external barrier 23 also has a partially circular development which follows the circumferential development of the rotating star-wheel 4.

More preferably, the upper barrier 21a, 21b comprises two upper circular rods 21a, 21b which are concentrically arranged with respect to a center of the rotating star-wheel 4. In particular, one of the upper circular rods 21a, which is referred to as "internal upper circular rod", has a radius of development which is shorter than the radius of development of the other of the upper circular rods 21b, which is referred to as "external upper circular rod".

Analogously, the lower barrier 22a, 22b comprises two lower circular rods 22a, 22b which are concentrically arranged with respect to the center of the rotating star-wheel 4. In particular, one of the lower circular rods 22a, which is referred to as "internal lower circular rod", has a radius of development which is shorter than the radius of development of the other of the lower circular rods 22b, which is referred to as "external lower circular rod".

In practice, the lids 200 are prevented to fall down thanks to the lower barrier 22a, 22b.

In addition, the lids 200 cannot raise out of the pre-established volume thanks to the upper barrier 21a, 21b.

In addition, the external barrier 23 prevents lateral movements of the lids 200 out of the pockets 5.

More preferably, the supporting means 20 further comprise an internal barrier 24 which is configured to abut the lids 200 so as to force their movement within the pockets 5. This allows to dynamically change the contact points of the lids 200 with the upper barriers 21a, 21b, the lower barriers 22a, 22b and the external barrier 23. Changing the contact points during the rotation of the star-wheel 4 allows to reduce shaded zones, thus contributing to achieve a more efficient and uniform sterilization of the lids 200.

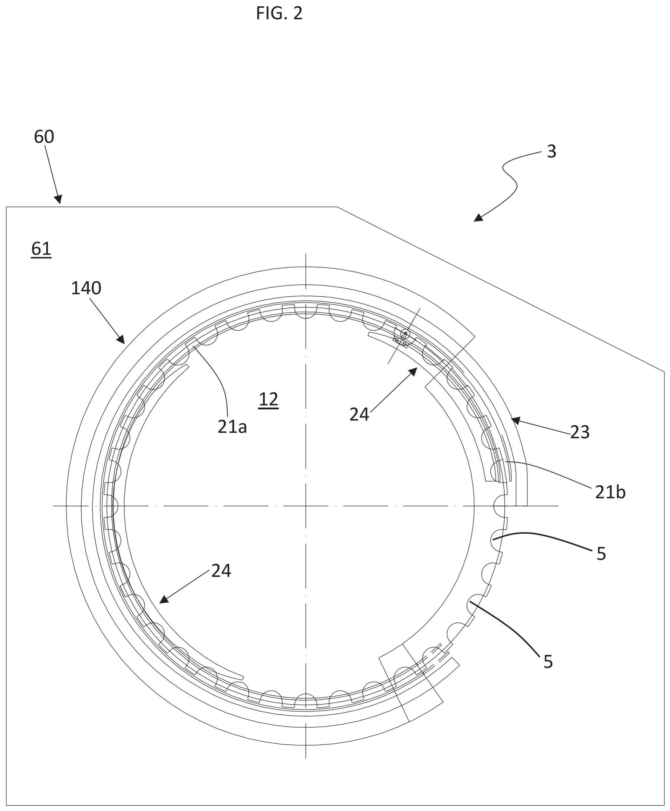

In particular, the internal barrier 24 has a partially circular development which follows the circumferential development of the rotating star-wheel 4. In the embodiment illustrated in figure 2, the internal barrier 24 has a shorter extension with respect to the upper barriers 21a, 21b, the lower barriers 22a, 22b and the external barrier 23. In particular, the internal barrier 24 is discontinuous, for example made by two separated arcuated portions.

Thanks to the internal barrier 24 each lid 200 is maintained within the corresponding pocket 5 in such a way that the lid 200 describes the linear trajectory in approaching the closing station 9.

A process for aseptically filling and closing cans is described hereafter. The process is carried out using the aseptic apparatus 1 just described. The cans 100 are sterilized, preferably in the sterilizing unit 110.

Then, the cans 100 are rinsed, preferably in the rinsing unit 120.

The cans 100, previously sterilized and rinsed, are filled with a product in the filling unit 2. In particular, transportation of cans 100 from the can sterilizing unit 110 to the filling unit 2 can be done either by star-wheels or by timing screw-worms.

In the meantime, the lids 200 start to be delivered to the rotating star-wheel 4 of the lid sterilizing unit 3. In particular, the lids 200 are delivered one after the other from the bottom of the stack in the longitudinal guide 7 of the lid infeed device 6.

The filled cans 100 exiting from the filling unit 2 are transported by the conveyor 8 towards the closing station 9. The cans 100 are made to advance along the linear advancing direction A-A.

The lids 200 are sterilized in the sterilizing unit 3, for example by spraying a sterilizing substance.

Preferably, each lid 200 is sterilized just before being applied on a corresponding can 100 or during the application to said can 100.

In particular, the sterilization of each lid 200 is at least partially overlapped to the step of applying the lid 200 onto the corresponding can 100 in the closing station 9.

In particular, each lid 200 is sterilized when it still held in a corresponding pocket 5 of the rotating star-wheel 4, while approaching the closing station 9.

As already explained, is the supporting means 20 for supporting the lids 200 are designed so as to reduce the contact points with the lids 200. This allows to better reach the lid 200 with the sterilizing substance.

As already said, the sterilization of the lids 200 may also occur by radiations.

Each sterilized lid 200 is deposited on the open top 100a of a can 100 arrived in the closing station 9.

This is done by pushing the sterilized lid 200 out of its pocket 5 and lowering the sterilized lid 200 onto said can 100. The sterilizing lid 200 is then placed and pressed on the corresponding can 100 so as to close its open top 100a.

According to one embodiment, the application of the sterilized lid 200 to the corresponding can 100 is done punctually, meaning that the lid 200 follows a circular trajectory due to the rotation of the rotating star-wheel 4 and meets the can 100 which is arriving linearly along the linear advancing direction A-A.

According to another embodiment of the invention, the application of the sterilized lid 200 to the corresponding can 100 is not punctual.

In this embodiment, the sterilized lid 200 follows a linear trajectory in approaching the closing station 9. The linear trajectory is parallel or tangent to the linear advancing direction A-A of the cans 100.

The cans 100 with the lids 200 applied thereon are then conveyed towards the seaming unit 10.

In the seaming unit 10 the lids 200 are crimped on the cans 100.

The process may further comprise a step of adjusting a height of the rotating star-wheel 4 with respect to the floor or ground G.

For example, this may be done at the beginning of the process or in case of a can format change.

The characteristics of the aseptic apparatus and process for filling and closing cans, according to the present invention, prove to be clearly indicated in the description provided.

In particular, the sterilization of the lids is performed in the star-wheel which dispenses the lids to the cans, thus achieving a more compact solution with respect to the prior art.

In addition, the sterilization effect is reliable and efficient since the lids are held in the pockets, thus they do not need to be separated for being sterilized, and then to be packed again to be fed to a lid dispenser.

In the claimed solution, the lids are sterilized just before being applied to the cans or during this application, which reduces the risks of recontamination with respect to the prior art.

The solution is easy since the cans are maintained at the same level during their advancement, whereas the sterilized lids are simply lowered by pushing organs.

## Claims

1. An aseptic apparatus (1) for filling and closing cans (100), the aseptic apparatus (1) comprising:
- a filling unit (2) configured to receive the cans (100) and to inject a product within the cans (100);
- a lid sterilizing unit (3) configured to sterilize lids (200) and comprising a rotating star-wheel (4) having a plurality of pockets (5), each pocket (5) of said plurality of pockets (5) being adapted to receive one of the lids (200);
- a conveyor (8) configured to advance the cans (100) filled with product from the filling unit (2) towards the lid sterilizing unit (3), said lid sterilizing unit (3) being configured to lower each of the sterilized lids (200) held in one of the pockets (5) on a corresponding can (100) laying on said conveyor (8) in a closing station (9) and to place and press said sterilized lid (200) on said corresponding can (100);
- isolating means (50, 60) within which the filling unit (2) and the sterilizing unit (3) are arranged;
- a seaming unit (10) arranged downstream the closing station (9) so as to receive the cans (100) with lids (200) applied thereon, said seaming unit (10) being located outside the isolating means (50, 60), said conveyor (8) extending toward said seaming unit (10) so that a first tract (8a) of said conveyor (8) is within the isolating means (50, 60) and a second tract (8b) of said conveyor (8) is outside the isolating means (50, 60).

2. The aseptic apparatus (1) according to claim 1, wherein the rotating star-wheel (4) of the lid sterilizing unit (3) is arranged in a protected environment (12) which is saturated with VHP.

3. The aseptic apparatus (1) according to claim 1 or 2, wherein said lid sterilizing unit (3) comprises supporting means (20) for supporting each lid (200) in a loose condition within a corresponding pocket (5) of the rotating star-wheel (4) in such a way that a circular trajectory of the lid (200) held in said loose condition in said pocket (5) is tangent in the closing station (9) to a line that is parallel to a linear advancing direction (A-A) of the cans (100) laying on the conveyor (8).

4. The aseptic apparatus (1) according to claim 1 or 2, wherein said lid sterilizing unit (3) comprises supporting means (20) for supporting each lid (200) in a loose condition within a corresponding pocket (5) of the rotating star-wheel (4) in such a way that the lid (200) held in said loose condition said pocket (5) describes a linear trajectory in approaching the closing station (9), said linear trajectory being parallel or tangent to a linear advancing direction (A-A) of the cans (100) laying on the conveyor (8).

5. The aseptic apparatus (1) according to claim 3 or 4, wherein said supporting means (20) comprise at least:
- an upper barrier (21a, 21b) configured to limit upper movements of each lid (200) held in the corresponding pocket (5);
- a lower barrier (22a, 22b) configured to limit lower movements of each lid (200) held in the corresponding pocket (5);
- an external barrier (23) configured to limit lateral movements of each lid (200) held in the corresponding pocket (5).

6. The aseptic apparatus (1) according to claim 5, wherein both the upper barrier (21a, 21b) and the lower barrier (22a, 22b) have a partially circular development which follows the circumferential development of the rotating star-wheel (4).

7. The aseptic apparatus (1) according to claim 6, wherein the upper barrier (21a, 21b) comprises two upper circular rods (21a, 21b) which are concentrically arranged with respect to a center of the rotating star-wheel (4), and the lower barrier (22a, 22b) comprises two lower circular rods (22a, 22b) which are concentrically arranged with respect to the center of the rotating star-wheel (4).

8. The aseptic apparatus (1) according to any one of the claims 5 to 7, wherein said supporting means (20) further comprise an internal barrier (24) configured to abut each lid (200) held in the corresponding pocket (5) so as to force the lid (200) to change its position.

9. The aseptic apparatus (1) according to any one of the preceding claims, wherein the lid sterilizing unit (3) comprises a pushing organ (16) configured to lower each lid (200) towards a corresponding can (100) laying on the conveyor (8) in the closing station (9).

10. The aseptic apparatus (1) according to any one of the claims 1 to 8, wherein the lid sterilizing unit (3) comprises a pushing organ integrally fixed to the rotating star-wheel (4) in correspondence of each pocket (5) of the plurality of pockets (5), each pushing organ being configured to lower a corresponding lid (200) towards a can (100) laying on the conveyor (8) in the closing station (9).

11. The aseptic apparatus (1) according to any one of the preceding claims, wherein the lid sterilizing unit (3) comprises dispensing means (31, 32, 33, 34) for dispensing a sterilizing substance, said dispensing means (31, 32, 33, 34) comprising first nozzles (31) located at a higher level with respect to the pockets (5) and second nozzles (32) located at a lower level with respect to the pockets (5), the first nozzles (31) and the second nozzles (32) being configured to dispense the sterilizing substance respectively towards top of the lids (200) and bottom of the lids (200).

12. The aseptic apparatus (1) according to any one of claims 1 to 10, wherein the lid sterilizing unit (3) is configured to sterilize the lids (200) by radiation.

13. The aseptic apparatus (1) according to any one of the preceding claims, further comprising actuating means configured to adjust a height of the rotating star-wheel (4) with respect to a floor or ground (G).

14. The aseptic apparatus (1) according to any one of the preceding claims, further comprising an upper guide (15) extending from the closing station (9) to the seaming unit (10) and configured to maintain the lids (200) placed and pressed on the corresponding cans (100) upon leaving the closing station (9).

15. The aseptic apparatus (1) according to claim 14, wherein a sterile air flow is established over the upper guide (15) in order to flow on the lids (200) applied on the advancing cans (100).

16. The aseptic apparatus (1) according to any one of the preceding claims, further comprising a lid infeed device (6) configured to supply the lids (200) to the pockets (5) of said rotating star-wheel (4), said lid infeed device (6) comprising a longitudinal guide (7) for housing the lids (200) in a stacked arrangement.

17. A process for aseptically filling and closing cans (100) using the aseptic apparatus (1) according to any one of the preceding claims, said process comprising the following steps:
- filling the cans (100) with a product in the filling unit (2);
- delivering lids (200) to the pockets (5) of said rotating star-wheel (4);
- sterilizing the lids (200) in the lid sterilizing unit (3);
- making the cans (100) to advance from the filling unit (2) towards the lid sterilizing unit (3) along a linear advancing direction (A-A) on said conveyor (8);
- for each can (100) arriving in the closing station (9), lowering one of the sterilized lids (200) onto said can (100), then placing and pressing the sterilized lid (200) on said can (100) so as to close it;
- conveying the cans (100) with the lids (200) applied thereon towards the seaming unit (10);
- crimping the lids (200) on the cans (100).

18. The process according to claim 17, wherein the sterilization of each lid (200) in the sterilizing unit (3) is at least partially overlapped to the step of placing lid (200) onto the corresponding can (100) in the closing station (9).

19. The process according to claim 17 or 18, wherein lowering each sterilized lid (200) comprises pushing the sterilized lid (200) out of the corresponding pocket (5).

20. The process according to any one of the claims 17 to 19, wherein each lid (200) on the rotating star-wheel (4) follows a circular trajectory that is tangent in the closing station (9) to a line that is parallel to the linear advancing direction (A-A) of the cans (100).

21. The process according to any one of the claims 17 to 20, wherein each lid (200) on the rotating star-wheel (4) follows a linear trajectory in approaching the closing station (9), the linear trajectory being parallel or tangent to the linear advancing direction (A-A) of the cans (100).

22. The process according to any one of the claims 17 to 21, further comprising sterilizing or washing the conveyor (8).

23. The process according to any one of the claims 17 to 22, further comprising adjusting a height of the rotating star-wheel (4) with respect to a floor or ground (G).
